(19) Europäisches Patentamt
European Patent Office
Office européen des brevets

(11) Veröffentlichungsnummer: **0 255 689 B1**

# (12) EUROPÄISCHE PATENTSCHRIFT

(45) Veröffentlichungstag der Patentschrift: **21.11.91**

(51) Int. Cl.5: **C07D 213/89**

(21) Anmeldenummer: **87110959.1**

(22) Anmeldetag: **29.07.87**

## (54) Verfahren zur Herstellung von 1-Hydroxy-2-pyridonen.

(30) Priorität: **02.08.86 DE 3626210**

(43) Veröffentlichungstag der Anmeldung:
**10.02.88 Patentblatt 88/06**

(45) Bekanntmachung des Hinweises auf die Patenterteilung:
**21.11.91 Patentblatt 91/47**

(84) Benannte Vertragsstaaten:
**AT BE CH DE ES FR GB GR IT LI LU NL SE**

(56) Entgegenhaltungen:
**EP-A- 0 241 918**
**DE-A- 1 795 270**
**DE-A- 2 214 608**

**A. WEISSBERGER: "The chemistry of heterocyclic compounds", 1960, Seiten 178-179,284-285, Interscience Publishers, Inc., New York, US**

(73) Patentinhaber: **HOECHST AKTIENGESELLSCHAFT**
**Postfach 80 03 20**
**W-6230 Frankfurt am Main 80(DE)**

(72) Erfinder: **Reuschling, Dieter, Dr.**
**Beethovenstrasse 27**
**W-6308 Butzbach(DE)**
Erfinder: **Gröbel, Bengt-Thomas, Dr.**
**Im Hainpfad 10**
**W-6232 Bad Soden am Taunus(DE)**

**EP 0 255 689 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

1-Hydroxy-2-pyridon ist die Verbindung der Formel I (s. Anspruch 1), in der $R^1$ und $R^2$ Wasserstoff sind, und Grundkörper einer Reihe wertvoller Substanzen mit antibakterieller und antimykotischer Wirksamkeit (vgl. US-PS 3 269 904, DE-PS 1 795 270 und DE-PS 2 214 608). Solche biologische wirksamen Substanzen sind z.B. 1-Hydroxy-4-methyl-6-(2,4,4-trimethylpentyl)-2-pyridon, 1-Hydroxy-4-methyl-6-cyclohexyl-2-pyridon und 1-Hydroxy-4-methyl-6-[4-(4-chlorphenoxy)-phenoxy-methyl]-2-pyridon.

Die 1-Hydroxy-2-pyridone werden zweckmäßig nach dem in der DE-PS 2 214 608 beschriebenen Verfahren durch Umsetzung der entsprechenden 2-Pyrone mit Hydroxylamin oder einem seiner Salze in Gegenwart eines - gegebenenfalls substituierten - Aminopyridins oder Imidazols hergestellt. Das Aminopyridin oder Imidazol wird hierbei vorteilhaft in wenigstens äquimolarer Menge, bezogen auf das Hydroxylammoniumsalz, eingesetzt. Als Temperaturbereich werden Temperaturen zwischen Raumtemperatur und 150° C, vorzugsweise zwischen 50 und 120° C angegeben. Bei der Umsetzung erfolgt ein Austausch des Ringsauerstoffatoms des 2-Pyrons durch die N-OH-Gruppe.

In dem auf die Herstellung des 1-Hydroxy-4-methyl-6-(2,4,4-trimethylpentyl)-2-pyridons gerichteten Beispiel (Nr. 9) der DE-PS 2 214 608 wird 4-Methyl-6-(2,4,4-trimethylpentyl)-2-pyron mit Hydroxylaminhydrochlorid in Gegenwart von 2-Aminopyridin 43 Stunden bei 70° C umgsesetzt, wobei dann nach üblicher Aufarbeitung eine Ausbeute von 67 % an dem gewünschten Pyridon erhalten wird. Dieses langwierige Verfahren hat den weiteren Nachteil, daß dabei beträchtliche Mengen an den relativ wertvollen und teuren Aminopyridinen und/oder Imidazolen verwendet werden müssen, die wegen ihres Wertes und auch aus Umweltschutzgründen nach beendeter Reaktion wieder zurückgewonnen werden müssen.

Es heißt zwar in der DE-PS 2 214 608, daß man einen Teil der wertvollen Aminverbindungen auch durch andere Säureakzeptoren organischer oder anorganischer Natur ersetzen kann, doch soll dieser Ersatz dann bisweilen zu einer merklichen Verlangsamung der Reaktion und auch zu einer Erschwerung der Rückgewinnung des Amins führen.

Es wurde nun gefunden, daß eine Verbesserung des bekannten Verfahrens ohne wesentliche Beeinträchtigung des Reaktionsergebnisses dadurch gelingt, daß man statt der Aminverbindungen Alkalicarbonate und/oder -hydrogencarbonate verwendet.

Gegenstand der Erfindung ist somit ein Verfahren zur Herstellung von 1-Hydroxy-2-pyridonen der allgemeinen Formel I (s. Patentanspruch 1) durch Umsetzung der Pyrone der Formel II (s. Patentanspruch 1) mit einem Hydroxylammoniumsalz in Gegenwart von basischen Verbindungen, das dadurch gekennzeichnet ist, daß man als basische Verbindungen Alkalicarbonate und/oder -hydrogencarbonate in einer Menge verwendet, die der des Hydroxylammoniumsalzes mindestens äquivalent ist und bei einer Temperatur von 50° bis 120° c arbeitet.

$R^1$ bedeutet einen, gegebenenfalls verzweigten, höchstens einfach olefinisch ungesättigten aliphatischen Kohlenwasserstoffrest mit 1 bis 17 Kohlenstoffatomen, einen Cycloalkylrest mit 3 bis 8, vorzugsweise 6 Kohlenstoffatomen im Ring, der noch 1 bis 2 Alkylgruppen mit jeweils 1 bis 3 C-Atomen, insbesondere Methyl tragen kann und direkt oder über eine Methylen- oder Äthylengruppe an den Pyridonring gebunden ist, einen unsubstituierten oder im aromatischen Kern durch 1 bis 3 Alkyl-, unsubstituierte oder substituierte Benzyl-, Alkoxy-, unsubstituierte oder substituierte Phenoxygruppen oder Halogenatome substituierten Phenyl-, Phenylalkylmethylrest und $R^2$ Wasserstoff, einen höchstens einfach olefinisch ungesättigten niedermolekularen (d.i. mit 1 bis 6 Kohlenstoffatomen) aliphatischen Kohlenwasserstoffrest oder einen Benzylrest. Als Substituent im Rest $R^1$ oder $R^2$ enthaltene Benzylgruppen können in gleicher Weise substituiert sein, wie es oben für die Phenylreste angegeben ist. Unter den Resten $R^1$ und $R^2$, die den Phenylkern enthalten, sind solche bevorzugt, in denen dieser Phenylkern nicht oder nur einmal oder zweimal substituiert ist. Bevorzugt unter den für $R^2$ genannten Resten sind Alkylreste mit 1 bis 4, insbesondere 1 oder 2 Kohlenstoffatomen, und unter den Alkenylresten solche mit 2, 3 oder 4 Kohlenstoffatomen.

Das Hydroxylammoniumsalz muß naturgemäß in äquimolaren Mengen, bezogen auf das umzusetzende 2-Pyron, eingesetzt werden; zur Beschleunigung der Reaktion kann es jedoch auch im Überschuß eingesetzt werden, wobei dann oft verbesserte Ausbeuten erhalten werden. Auch kann es zweckmäßig sein, das Hydroxylaminsalz im mehreren Portionen im Verlauf der Reaktion zuzusetzen.

Als Hydroxylammoniumsalze können im Prinzip alle möglichen Salze des Hydroxylamins, z.B. das Chlorid, das Sulfat, das Acetat usw. verwendet werden. Bevorzugt ist jedoch die Durchführung der Umsetzung mit dem leicht zugänglichen Hydroxylammoniumsulfat.

Als Alkalicarbonate und -hydrogencarbonate kommen praktisch alle Carbonate und Bicarbonate der Alkalimetalle in Betracht, also z.B. $Li_2CO_3$, $Na_2CO_3$, $K_2CO_3$, $NaHCO_3$, $KHCO_3$. Bevorzugt sind die Carbonate und Bicarbonate des Natriums und Kaliums; besonders bevorzugt ist $Na_2CO_3$. Die Alkalicarbona-

te und -bicarbonate können sowohl einzeln als auch in praktisch beliebiger Mischung zum Einsatz gelangen. Ihre Menge ist zweckmäßig der verwendeten Menge an Hydroxylammoniumsalz mindestens äquivalent. Pro Mol Hydroxylammoniumchlorid ist beispielsweise mindestens 1/2 Mol $Na_2CO_3$ oder 1 Mol $NaHCO_3$ zu verwenden.

Zur Durchführung des erfindungsgemäßen Verfahrens mischt man vorteilhaft das 2-Pyron mit dem Hydroxylammoniumsalz und dem Alkalicarbonat und/oder -hydrogencarbonat und erwärmt den erhaltenen Kristallbrei, bis kein Pyron mehr nachzuweisen ist; das entstandene 2-Pyridon kann nach Abtrennung der anorganischen Salze direkt - oder besser noch als Salz einer organischen Base, z.B. als Äthanolaminsalz - isoliert werden.

Der Temperaturbereich, in welchem das erfindungsgemäße Verfahren ausgeführt wird, ist wesentlich enger als der für das Verfahren gemäß DE-PS 2 214 608. Die Höchsttemperatur sollt 120° C keinesfalls überschreiten. Die Reaktionstemperatur liegt zweckmäßig über 50 und vorzugsweise zwischen etwa 60 und 105° C.

Es ist auch möglich, inerte Lösungs- bzw. Verdünnungsmittel zuzusetzen. Im allgemeinen ist dies jedoch nicht erforderlich, aber bevorzugt. Dies steht im Gegensatz zum Verfahren der DE-PS 2 214 608, bei dem in keinem der 14 Beispiele solche Lösungs- bzw. Verdünnungsmittel mitverwendet worden sind. Bei dem erfindungsgemäßen Verfahren werden die Lösungs- bzw. Verdünnungsmittel im allgemeinen, wenn überhaupt, nur in kleinen Mengen, meistens bis zu 50 Gew.-% des gesamten Reaktionsansatzes, zugesetzt. Bevorzugt beträgt die Menge 3 bis 15 Gew.-%.

Die Lösungs- oder Verdünnungsmittel können polar oder unpolar, mit Wasser mischbar oder nicht mischbar sein. Z.B. können folgende Substanzen verwendet werden: Wasser, niedermolekulare Alkohole wie Methanol, Äthanol, Isopropanol, Äthylenglykol, Äthylenglykol-monomethyläther und Propylenglykol, Säureamide wie Dimethylformamid und Diäthylformamid und Ester wie Äthylacetat, Äther wie Diisopropyläther, chlorierte Kohlenwasserstoffe wie Chlorbenzol, Nitrile wie Acetonitril, Kohlenwasserstoffe aliphatischer, cycloaliphatischer oder aromatischer Natur.

Gegenüber dem Verfahren der DE-PS 2 214 608 zeichnet sich das erfindungsgemäße Verfahren infolge des Ersatzes der teuren Aminverbindungen durch die billigen und wohlfeilen Alkalicarbonate und -hydrogencarbonate nicht nur durch eine erhöhte Wirktschaftlichkeit aus, sondern durch die besonderen Vorteile, daß auf die basischen, die Umwelt belastenden Verbindungen und deren Wiedergewinnung und vollständige Entfernung aus dem Reaktionsansatz nach der Reaktion verzichtet werden kann und daß teilweise auch kürzere Reaktionszeiten erreicht werden. Dabei ist zu berücksichtigen, daß eine Wiedergewinnung der genannten basischen Verbindungen nur unter Inkaufnahme erheblicher Verluste möglich war, was zu weiteren Umweltbelastungen führte. Etwas verringerte Ausbeuten beeinträchtigen die erhöhte Wirtschaftlichkeit des Verfahrens nicht ernsthaft, da ja der Aufwand für die Aminverbindungen und die bei deren Verwendung in Kauf zu nehmenden Verluste bei dem vorliegenden Verfahren nicht eintritt.

Die Erfindung wird durch die folgenden Beispiele erläutert.

## Beispiele

1-9) Herstellung von 1-Hydroxy-4-methyl-6-(2,4,4-trimethylpentyl)-2-pyridon (Äthanolaminsalz)

1) 33,5 g (150 mMol) 4-Methyl-6-(2,4,4-trimethylpentyl)-2-pyron (99 %ig), 29,8 g (429 mMol) Hydroxylammoniumchlorid und 22,7 g (214,5 mMol ) Natriumcarbonat werden vereinigt und unter Rühren 8 Stunden auf 95° C erwärmt. Anschließend gibt man 100 ml Äthylacetat hinzu und kühlt auf Raumtemperatur. Die Festprodukte werden abgesaugt und mit 240 ml Äthylacetat gewaschen. Die Äthylacetat-Phase wird nach dem Abdestillieren (Destillatmenge 100 ml) bei ca. 50° C mit 8,5 ml (143 mMol) Äthanolamin versetzt. Nach dem Animpfen läßt man die Lösung zur Kristallisation abkühlen. Die Kristalle werden abgesaugt, mit wenig kaltem Äthylacetat gewaschen und getrocknet. Die Ausbeute an 1-Hydroxy-4-methyl-6-(2,4,4-trimethylpentyl)-2-pyridon (Äthanolamin-Salz) beträgt 24,6-26,8 g (55-60 %). Schmp. 123-6° C.

2) An Stelle von Hydroxylammoniumchlorid verwendet man bei sonst gleicher Arbeitsweise 35,2 g (214,6 mMol) Hydroxylammoniumsulfat; sonst wie Beispiel 1. Ausbeute 25-26,9 g (56-60,1 %). Schmp. 123-6° C.

3-7) 447 g (2 Mol) 4-Methyl-6-(2,4,4--trimethylpentyl)-2-pyron (99 %ig), 479 g (2,86 Mol) Hydroxylammoniumsulfat (98 %ig), 306,2 g (2,86 Mol) Natriumcarbonat (99 %ig) und ein Lösungs- bzw. Verdünnungsmittel (s. Tabelle) werden vereinigt und unter Rühren 20 Stunden auf 95° C ervärmt. Die noch warme Lösung versetzt man mit 2000 ml Äthylacetat, saugt von der Festsubstanz ab und wäscht mit 1000 ml Äthylacetat nach. Anschließend wird das Filtrat zweimal mit je 500 ml Wasser ausgerührt. Zur Trocknung

wird von der Äthylacetatlösung 1000 ml Äthylacetat abdestilliert. Danach versetzt man den Destillationsrückstand (Reaktionsprodukt + 2000 ml Äthylacetat) bei ca. 50 °C in den Beispielen 3 und 5 bis 7 mit 113 ml (= 1,89 Mol) Äthanolamin und im Beispiel 4 mit 143 ml (2,39 Mol) Äthanolamin; nach dem Animpfen läßt man die Lösung zur Kristallisation abkühlen. Die Kristalle werden abgesaugt, mit 200 ml kaltem Äthylacetat gewaschen und getrocknet.

| Beispiel | | Lösungs- bzw. Verdünnungsmittel (g) | [Mol] | $C_{14}H_{23}NO_2$·Äthanolamin [%] |
|---|---|---|---|---|
| 3 | Toluol | 410 | 5 | 49,5 |
| 4 | HO-Pyd.[a] | 118,7 | 0,5 | 50[b] |
| 5 | $H_2O$ | 4,5 | 0,25 | 54 |
| 6 | $H_2O$ | 18 | 1 | 53 |
| 7 | $H_2O$ | 36 | 2 | 53,5 |

a) HO-Pyd.= 1-Hydroxy-4-methyl-6-(2,4,4-trimethylpentyl)-2-pyridon

b) nach Abzug von 0,5 Mol, die als Lösungsmittel verwendet wurden.

8) 447 g (2 Mol) 4-Methyl-6-(2,4,4-trimethylpentyl)-2-pyron (99 %ig), 119,7 g (0,71 Mol) Hydroxylammoniumsulfat (98 %ig), 4,5 ml (0,25 Mol) Wasser und 306,2 g (2,86 Mol) Natriumcarbonat (99 %ig) werden vereinigt und unter Rühren auf 95 °C erwärmt. Im 60-minütigen Abstand werden 3 weitere Portionen von je 119,7 g (0,71 Mol) Hydroxylammoniumsulfat zugegeben. Insgesamt wird 20 Stunden bei 95 °C gerührt. Die noch warme Lösung arbeitet man wie im Beispiel 3 auf. Ausbeute 346,1 g (58 %); Schmelzpunkt 124,7 °C.

9A) 447 g (2 Mol) 4-Methyl-6-(2,4,4-trimethylpentyl)-2-pyron (99 %ig), 83,7 g (0,5 Mol) Hydroxylammoniumsulfat (98 %ig), 4,5 ml (0,25 Mol) Wasser und 214,1 g (2 Mol) Natriumcarbonat (99 %ig) werden vereinigt und unter Rühren auf 95 °C erwärmt. Im 60-minütigen Abstand werden 3 weitere Portionen von je 83,7 g (0,5 Mol) Hydroxylammoniumsulfat zugegeben. Insgesamt wird 20 Stunden bei 95 °C gerührt. Die Aufarbeitung erfolgt wie im Beispiel 3. Ausbeute 343 g (57,5 %); Schmelzpunkt 123-6 °C.

9B) Isolierung des 1-Hydroxy-4-methyl-6-(2,4,4-trimethylpentyl)-2-pyridons aus seinem Äthanolamin-Salz: 298,4 g (1 Mol) 1-Hydroxy-4-methyl-6-(2,4,4-trimethylpentyl)-2-pyridon-Äthanolaminsalz werden in 700 ml Äthylacetat aufgeschlämmt und bei Raumtemperatur mit 1,1 Mol 2,5 n Salzsäure versetzt. Nach kurzer Zeit erhält man eine klare Lösung. Die Äthylacetat-Phase wird sorgfältig abgetrennt und ohne weitere Behandlung direkt eingedampft. Als Rückstand erhält man das 1-Hydroxy-4-methyl-6-(2,4,4-trimethylpentyl)-2-pyridon als weißes kristallines Pulver. Ausbeute 234 g (98,6 %); Schmelzpunkt 107-110 °C.

10-13) Herstellung von 1-Hydroxy-4-methyl-6-cyclohexyl-2-pyridon-(Äthanolaminsalz)

10+11) Je 192 g (1 Mol) 4-Methyl-6-cyclohexyl-2-pyron, 239,5 g (1,43 Mol) Hydroxylammoniumsulfat und 153,1 g (1,43 Mol) Natriumcarbonat werden einmal mit 30 ml Toluol (Beispiel 10) bzw. 30 ml Methylcyclohexan (Beispiel 11) vermischt, langsam auf 90-95 °C erwärmt und 8 Stunden bei dieser Temperatur gehalten. Noch warm versetzt man mit 1000 ml Äthylacetat, saugt von der Festsubstanz ab und wäscht mit 500 ml Äthylacetat nach. Anschließend wird das Filtrat mit gesättigter Natriumbicarbonatlösung säurefrei gewaschen. Zur Trocknung werden von der Äthylacetatlösung 500 ml Äthylacetat abdestilliert. Danach versetzt man den Destillationsrückstand bei ca. 50 °C mit 56,6 ml (0,94 Mol) Äthanolamin; nach dem Animpfen läßt man die Lösung zur Kristallisation abkühlen. Die Kristalle werden abgesaugt, mit 100 ml kaltem Äthylacetat gewaschen und getrocknet. Die Ausbeute an 1-Hydroxy-4-methyl-6-cyclohexyl-2-pyridon (Äthanolamin-Salz) beträgt im Beispiel 10 134-142 g (50-53 %) und im Beispiel 11 134-139,5 g (50-56 %); Schmelzpunkt 138-142 °C.

12) 96 g (0,5 Mol) 4-Methyl-6-cyclohexyl-2-pyron, 29,9 g (0,179 Mol) Hydroxylammoniumsulfat und 19,1 g (0,179 Mol) Natriumcarbonat werden auf 90-95° C erwärmt. Nach 60 Minuten und danach im Abstand von je 60 Minuten werden insgesamt fünf weitere Portionen von 14,95 g (89,5 mMol) Hydroxylammoniumsulfat und 9,6 g (89,5 mMol) Natriumcarbonat zugesetzt. Nach der letzten Zugabe wird noch 90 Minuten bei 90-95° C gerührt. Die Aufarbeitung erfolgt analog Beispiel 10. Ausbeute 137 g (51 %); Schmelzpunkt 137-140° C.

13A) 96 g (0,5 Mol) 4-Methyl-6-cyclohexyl-2-pyron, 119,7 g (0,715 Mol) Hydroxylammoniumsulfat und 76,6 g (0,715 Mol) Natriumcarbonat werden 6,5 Stunden auf 95° C erwärmt. Nach dem Abkühlen nimmt man die Reaktionsmichung in 500 ml Wasser und 500 ml Äthylacetat auf, trennt die organische Phase ab und wäscht sie mit gesättigter Natriumhydrogencarbonatlösung säurefrei. Anschließend wird die organische Phase über Natriumsulfat getrocknet und das Filtrat bei ca. 50° C mit 28,3 ml (0,47 Mol) Äthanolamin versetzt. Die beim Abkühlen kristallisierende Substanz wird abgesaugt, mit 50 ml kaltem Äthylacetat gewaschen und getrocknet. Ausbeute 139 g (52 %); Schmelzpunkt 139-142° C.

13B) Isolierung des 1-Hydroxy-4-methyl-6-cyclohexyl-2-pyridons aus seinem Äthanolamin-Salz:
268,4 g (1 Mol) 1-Hydroxy-4-methyl-6-cyclohexyl-2-pyridon-Äthanolaminsalz werden in 700 ml Äthylacetat aufgeschlämmt und bei Raumtemperatur mit 1,1 Mol 2,5n Salzsäure versetzt. Nach kurzer Zeit erhält man eine klare Lösung. Die Äthylacetat-Phase wird sorgfältig abgetrennt und ohne weitere Behandlung direkt eingedampft. Als Rückstand erhält man das 1-Hydroxy-4-methyl-6-cyclohexyl-2-pyridon als weißes kristallines Pulver. Ausbeute 203 g (98 %); Schmelzpunkt 143° C.

## Patentansprüche

1. Verfahren zur Herstellung von 1-Hydroxy-2-pyridonen der Formel I

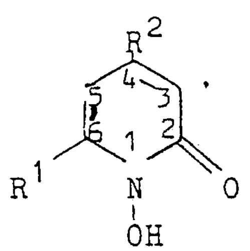

(I) ,

worin bedeuten
$R^1$ einen höchstens einfach olefinisch ungesättigten aliphatischen Kohlenwasserstoffrest mit 1 bis 17 Kohlenstoffatomen, einen Cycloalkylrest mit 3 bis 8 Kohlenstoffatomen im Ring, der noch 1 bis 2 Alkylgruppen mit jeweils 1 bis 3 C-Atomen tragen kann und direkt oder über eine Methylen- oder Äthylengruppe an den Pyridonring gebunden ist, einen unsubstituierten oder im aromatischen Kern durch 1 bis 3 Alkyl-, Benzyl-, Alkoxy-, Phenoxy- oder Halogenatome substituierten Phenyl-, Phenylalkylrest, wobei auch als Substituent enthaltene Benzyl- oder Phenoxygruppen in gleicher Weise substituiert sein können, $R^2$ Wasserstoff, einen höchstens einfach olefinisch ungesättigten aliphatischen Kohlenwasserstoffrest mit 1 bis 6 Kohlenstoffatomen oder einen Benzylrest, der unsubstituiert ist oder in der bei $R^1$ angegebenen Weise substituiert sein kann,
durch Umsetzung eines Pyrons der Formel II

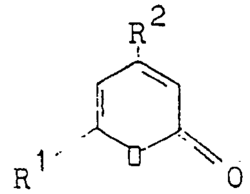

(II)

mit einem Hydroxylammoniumsalz in Gegenwart von basischen Verbindungen, dadurch gekennzeichnet, daß man als basische Verbindungen Alkalicarbonate und/oder -hydrogencarbonate in einer Menge verwendet, die der des Hydroxylammoniumsalzes mindestens äquivalent ist und bei einer Temperatur

von 50° bis 120° C arbeitet.

2. Verfahren nach Anspruch 1, dadurch gekennzeichnet, daß der Cycloalkylrest ein Cyclohexylrest ist.

3. Verfahren nach Anspruch 1 oder 2, dadurch gekennzeichnet, daß der Cycloalkylrest als Substituent noch Methyl enthält.

4. Verfahren nach einem oder mehreren der Ansprüche 1 bis 3, dadurch gekennzeichnet, daß $R^2$ höchstens 4 Kohlenstoffatome enthält.

5. Verfahren nach einem oder mehreren der Ansprüche 1 bis 4, dadurch gekennzeichnet, daß $R^2$ 1 oder 2 Kohlenstoffatome enthält.

6. Verfahren nach Anspruch 1, 4 oder 5, dadurch gekennzeichnet, daß Reste $R^1$ und $R^2$, die den Phenylkern enthalten, unsubstituiert sind oder höchstens 2 Substituenten tragen.

7. Verfahren nach einem oder mehreren der Ansprüche 1 bis 6, dadurch gekennzeichnet, daß als Hydroxylammoniumsalz das Hydroxylammoniumsulfat verwendet wird.

8. Verfahren nach einem oder mehreren der Ansprüche 1 bis 7, dadurch gekennzeichnet, daß als Alkalicarbonate oder -hydrogencarbonate die des Natriums oder Kaliums verwendet werden.

9. Verfahren nach einem oder mehreren der Ansprüche 1 bis 8, dadurch gekennzeichnet, daß als Alkalicarbonat Natriumcarbonat verwendet wird.

10. Verfahren nach einem oder mehreren der Ansprüche 1 bis 9, dadurch gekennzeichnet, daß bei einer Temperatur von 60° bis 105° C gearbeitet wird.

11. Verfahren nach einem oder mehreren der Ansprüche 1 bis 10, dadurch gekennzeichnet, daß in Gegenwart eines Lösungs-oder Verdünnungsmittels gearbeitet wird.

12. Verfahren nach einem oder mehreren der Ansprüche 1 bis 11, dadurch gekennzeichnet, daß in Gegenwart eines Lösungs-oder Verdünnungsmittels gearbeitet wird, das in einer Menge bis zu 50 Gew.-% des gesamten Reaktionsansatzes zugegen ist.

13. Verfahren nach einem oder mehreren der Ansprüche 1 bis 12, dadurch gekennzeichnet, daß in Gegenwart eines Lösungs-oder Verdünnungsmittels gearbeitet wird, das in einer Menge von 3 bis 15 Gew.-% des gesamten Reaktionsansatzes zugegen ist.

## Claims

1. A process for the preparation of 1-hydroxy-2-pyridones of the formula I

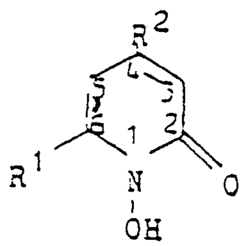

(1/I),

in which
$R^1$ denotes an at most olefinically monounsaturated aliphatic hydrocarbon radical having 1 to 17 carbon atoms, a cycloalkyl radical having 3 to 8 carbon atoms in the ring and which can carry in addition 1 to 2 alkyl groups in each case having 1 to 3 carbon atoms and which is bonded to the pyridone ring either directly or via a methylene or ethylene group, or a phenyl or phenylalkyl radical which is unsubstituted

or substituted in the aromatic ring by 1 to 3 alkyl, benzyl, alkoxy, phenoxy or halogen atoms, where the benzyl or phenoxy groups included as substituent may be substituted in the same fashion, $R^2$ denotes hydrogen, an at most olefinically monounsaturated aliphatic hydrocarbon radical having 1 to 6 carbon atoms or a benzyl radical which is unsubstituted or may be substituted in the same fashion as in the case of $R^1$, by reacting a pyrone of the formula II

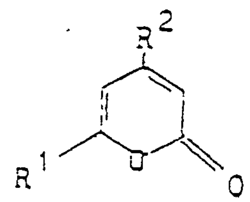

(1/II)

with a hydroxylammonium salt in the presence of basic compounds, wherein the basic compounds used are alkali metal carbonates and/or hydrogen carbonates in an amount which is at least equivalent to that of the hydroxylammonium salt and the reaction is carried out at a temperature of 50° to 120° C.

2. The process as claimed in claim 1, wherein the cycloalkyl radical is a cyclohexyl radical.

3. The process as claimed in claim 1 or 2, wherein the cycloalkyl radical contains, in addition, methyl as substituent.

4. The process as claimed in one or more of claims 1 to 3, wherein $R^2$ contains at most 4 carbon atoms.

5. The process as claimed in one or more of claims 1 to 4, wherein $R^2$ contains 1 or 2 carbon atoms.

6. The process as claimed in claim 1, 4 or 5, wherein radicals $R^1$ and $R^2$ which contain a phenyl ring are unsubstituted or carry at most 2 substituents.

7. The process as claimed in one or more of claims 1 to 6, wherein the hydroxylammonium salt used is hydroxylammonium sulfate.

8. The process as claimed in one or more of claims 1 to 7, wherein the alkali metal carbonates or hydrogen carbonates used are those of sodium or potassium.

9. The process as claimed in one or more of claims 1 to 8, wherein the alkali metal carbonate used is sodium carbonate.

10. The process as claimed in one or more of claims 1 to 9, wherein the process is carried out at a temperature from 60 to 105° C.

11. The process as claimed in one or more of claims 1 to 10, wherein the process is carried out in the presence of a solvent or diluent.

12. The process as claimed in one or more of claims 1 to 11, wherein the process is carried out in the presence of a solvent or diluent, which is present in an amount up to 50% by weight of the total reaction batch.

13. The process as claimed in one or more of claims 1 to 12, wherein the process is carried out in the presence of a solvent or diluent, which is present in an amount from 3 to 15% by weight of the total reaction batch.

**Revendications**

1. Procédé pour préparer des 1-hydroxy-2-pyridinones de formule I :

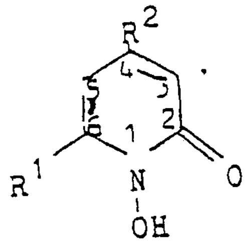

(I) ,

dans laquelle
$R^1$ représente un radical hydrocarboné aliphatique, à insaturation au plus monoéthylénique, comportant 1 à 17 atomes de carbone, un radical cycloalkyle comportant 3 à 8 atomes de carbone dans le cycle, qui peut également porter 1 à 2 groupes alkyle comportant chacun 1 à 3 atomes de carbone et qui est lié au cycle pyridinone directement ou par l'intermédiaire d'un groupe méthylène ou éthylène, un radical phényle ou phénylalkyle, substitué ou non sur le noyau aromatique par 1 à 3 groupes alkyle, benzyle, alcoxy, phénoxy ou atomes d'halogène, les groupes benzyle ou phénoxy contenus comme substituants pouvant être substitués de la même façon, $R^2$ représente un atome d'hydrogène, un radical hydrocarboné aliphatique, à insaturation au plus monoéthylénique, comportant 1 à 6 atomes de carbone, ou un radical benzyle, qui peut être substitué ou non de la façon mentionnée pour $R^1$,

par réaction d'une pyrone de formule II:

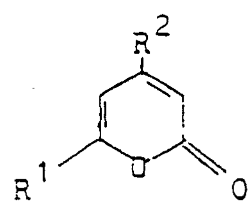

(II)

avec un sel d'hydroxylammonium, en présence de composés basiques, caractérisé en ce que l'on utilise, comme composés basiques, des carbonates et/ou des hydrogénocarbonates alcalins, en une quantité au moins équivalente à celle du sel d'hydroxylammonium, et on opère à une température de 50 à 120 °C.

2. Procédé selon la revendication 1, caractérisé en ce que le radical cycloalkyle est un radical cyclohexyle.

3. Procédé selon la revendication 1 ou 2, caractérisé en ce que le radical cycloalkyle contient également le groupe méthyle comme substituant.

4. Procédé selon une ou plusieurs des revendications 1 à 3, caractérisé en ce que $R^2$ contient au plus 4 atomes de carbone.

5. Procédé selon une ou plusieurs des revendications 1 à 4, caractérisé en ce que $R^2$ contient 1 ou 2 atomes de carbone.

6. Procédé selon la revendication 1, 4 ou 5, caractérisé en ce que les radicaux $R^1$ et $R^2$ qui contiennent le noyau phényle sont non substitués ou portent au plus 2 substituants.

7. Procédé selon une ou plusieurs des revendications 1 à 6, caractérisé en ce que l'on utilise le sulfate d'hydroxylammonium comme sel d'hydroxylammonium.

8. Procédé selon une ou plusieurs des revendications 1 à 7, caractérisé en ce que l'on utilise, comme carbonates ou hydrogénocarbonates alcalins, ceux de sodium ou de potassium.

9. Procédé selon une ou plusieurs des revendications 1 à 8, caractérisé en ce que l'on utilise le carbonate de sodium comme carbonate alcalin.

10. Procédé selon une ou plusieurs des revendications 1 à 9, caractérisé en ce que l'on opère à une température de 60 à 105° C.

11. Procédé selon une ou plusieurs des revendications 1 à 10, caractérisé en ce que l'on opère en présence d'un solvant ou d'un diluant.

12. Procédé selon une ou plusieurs des revendications 1 à 11, caractérisé en ce que l'on opère en présence d'un solvant ou d'un diluant, présent en une quantité allant jusqu'à 50% en poids, par rapport au poids total du milieu réactionnel.

13. Procédé selon une ou plusieurs des revendications 1 à 12, caractérisé en ce que l'on opère en présence d'un solvant ou d'un diluant, présent en une quantité de 3 à 15% en poids, par rapport au poids total du milieu réactionnel.